Europäisches Patentamt

European Patent Office

Office européen des brevets

(19)

(11) Publication number: **0 387 586**
**A1**

(12)

# EUROPEAN PATENT APPLICATION

(21) Application number: 90103734.1

(22) Date of filing: 26.02.90

(51) Int. Cl.⁵: **A01N 63/00, D06M 13/355,**
**C09D 5/14, C09D 5/16,**
**D21H 21/36, A61L 15/46**

(30) Priority: 27.02.89 JP 43234/89

(43) Date of publication of application:
**19.09.90 Bulletin 90/38**

(84) Designated Contracting States:
**CH DE ES FR GB IT LI**

(71) Applicant: **SAKAI ENGINEERING CO., LTD.**
**No. 15-1, Hanandonaka 2-chome**
**Fukui-shi, Fukui-ken(JP)**

Applicant: **MEIJI SEIKA KAISHA LTD.**
**4-16 Kyobashi 2-chome**
**Chuo-ku Tokyo(JP)**

(72) Inventor: **Katayama, Matsuko**
**No. 202-13, Maehama, Oshimacho**
**Fukui-shi, Fukui-ken(JP)**
Inventor: **Yasuda, Kimiaki**
**No. 18-2, Bynkyo 1-chome**
**Fukui-shi, Fukui-ken(JP)**
Inventor: **Takabe, Hidehi, c/o Meiji Seika**
**Kaisha Ltd.**
**Pharmaceutical Research Center 760**
**Morooka-cho**
**Kohoku-ku Yokohama-shi Kanagawa-ken(JP)**
Inventor: **Fukatsu, Shunzo c/o Meiji Seika**
**Kaisha Ltd.**
**Pharmaceutical Research Center 760**
**Morooka-cho**
**Kohoku-ku Yokohama-shi Kanagawa-ken(JP)**

(74) Representative: **Patentanwälte Deufel- Schön-**
**Hertel- Lewald- Otto**
**Isartorplatz 6**
**D-8000 München 2(DE)**

(54) **Antimicrobial/antibromic processing preparations.**

(57) Amino-glycosidic antibiotics are applied as antimicrobial antibromic processing preparations.

# ANTIMICROBIAL/ANTIBROMIC PROCESSING PREPARATIONS

## BACKGROUND OF THE INVENTION

### Field of the Invention

The present invention relates to a technique which makes it possible to prevent resource deteriorations attributable to microbes by the application of amino-glycosidic antibiotics to the antimicrobial/antibromic processings of fiber products, packaging materials, industrial materials and so on.

### Prior Art and Problems

Although microbes make a great contribution to human life, harmful microbes not only deteriorate most of the substances having relation to food, clothing and housing but also much damage in every aspect of human life, inclusive of environment. It is thus a long-standing problem of vital importance to protect human life against the damage caused by such harmful microbes.

Heretofore, various methods for effectively controlling harmful microbes have been studied. Of these, one effective method makes use of microbicides capable of killing off harmful microbes within a very short time. However, most of the currently available microbicides are synthetic microbicides of high toxicity. In addition, a problem of residual toxicity is unavoidable because of the likeliness that microbicides may be entrained direct in the substance to be treated or scattered in environment.

However, studies have recently been made with a view to decrease the toxicity of microbicides. Thus, intensive studies have now been focused on the development of microbicides acting specifically upon bacteria and reducing their percutaneous and oral absorption down to much lower levels or of immobilized microbicides which are insoluble and show little or no toxicity. In particular, the slow releasability and insolubility of microbicides have been studied so as to enhance the antimicrobial fastness of materials to which antimicrobial properties are imparted. Depending upon the purposes, the slow releasing microbicides and immobilized microbicides (insoluble microbicides) may be used. It is only microbicides having active moieties found on their surface layers that can possibly show microbicidal activity even when immobilized. Such microbicides having active moieties on their surface layers are primarily represented by an antibiotic polymyxin, cationic surfactants, amphoteric surfactants, biguanides, higher fatty acids and phenols. Of these compounds, quaternary ammonium salts, a betaine type of amphoteric surfactants, biguanides and a hydrophobic compound having a long-chain alkyl group have been reported to provide immobilized microbicides.

Because of not being likely to elute, the immobilized microbicides have the advantages of being neither entrained direct in the substance to be treated nor scattered in environment. However, their use is limited, since if organic matters are deposited onto a surface smeared with the microbicides, there is then a sharp deterioration of their antimicrobial properties. On the other hand, the slow-releasing microbicides have the advantages of producing a sustained antimicrobial effect and being of diverse types. Yet the problem of residual toxicity remains unsolved.

According to the present invention, there is provided a technique for permitting an amino-glycosidic antibiotic to be applied as a slow-releasing microbicide for antimicrobial/antibromic processings. What underlies the present invention is that such a microbicide has the properties as mentioned below:

The microbicide according to the present invention is:

(1) highly specific to cells, of much lower toxicity to human beings and scattered in environment in a much smaller amount, when comparing with other microbicides, because it can prevent any proliferation of microbes in a very small amount;

(2) safely used for the weakly aged and infants because of being endermically or intestinally absorbed in only small amounts;

(3) of thermal stability so high that it is unlikely to be denatured by heat treatments in the processing steps for various materials; and

(4) of high cost performance.

## DETAILED EXPLANATION OF THE INVENTION

As to how this microbicide produces an antimicrobial action, it is presumed that it is deposited onto bacterial ribosomes to limit their working and, consequently, prevent their protein synthesis. Now, more than 100 amino-glycosidic antibiotics are found. Of these, streptomycin, neomycin, paromomycin, kanamycin, kanamycin B, gentamycin C, ribostamycin, tobramycin, spectinomycin, sisomycin, astromycin and so on are used as chemotherapeutic preparations in Japan. Dibekacin, amikacin, netylmycin, etc. obtained by the chemical inducement of the above substances on the basis of their resistance working are also widely used as chemotherapeutic preparations. In addition, kasugamycin and varidamycin A are used as disease controllers exclusively for agricultural purposes, while hygromycin B and destomycin A are empolyed as vermicides exclusively for livestock and poultry.

With the microbicidal preparation of the present invention and a commercially available antimicrobial preparation, antimicrobial processings were applied to fibers and corrugated cardboards for the comparison of the results and costs. As the materials to be processed, use was made of a mixed fabric consisting of 65 % of cotton and 35 % of polyester and a corrugated cardboard not subjected to any surface treatment.

As the amino-glycosidic antibiotic, a commercially available ribostamycin preparation was used with a quaternary ammonium salt octa-decyl dimethyl (3-trimethoxysilyl) propyl ammonium chloride, and organosilicone was used as the crosslinker. By this antimicrobial processing, the ribostamycin and quaternary ammonium salt were chemically bonded to the surface of the fiber or corrugated cardboards, while using the organosilicone as the medium. Due to their low reactivity, the ribostamycin and quaternary ammonium salt per se are only deposited on the surface of the material to be processed, rather than being chemically bonded thereto. Hence, they dissolve in water by washing, etc. and so cannot provide a sustained antimicrobial effect to the material over an extended period of time. The organo-silicone has a plurality of functional groups, a part of which is coupled to a first substance and another part of which is coupled to a third substance, whereby the second and third substances are crosslinked together.

For antimicrobial assay, Staphylococcus aureus (IFO 12732 strain) was used. Testing was carried out under the following conditions. To obtain antimicrobial processing preparations, the ribostamycin and quaternary ammonium salt were each added to a 2 % solution of the organosilicone (C-200 manufactured by Nikka Kagaku Co., Ltd.) at a dilution stage from a concentration of 1 % to a concentration of $2^{-7}$ times.

In antimicrobial processing testing in which a corrugated cardboard was used as the testing material, a given concentration of the antimicrobial processing preparation adjusted to a starchy color was well-stirred and then hand-smeared on one side of a 10 cm long and 20 cm wide corrugated cardboard test piece. After hot-air drying, the testing piece was smeared on one side with a Staphylococcus aureus- proliferating liquid medium containing 0.25 % of agar over 24 hours, and was then dried at room temperature for subsequent 48-hour culture at 30° C.

For the antimicrobial processing treatment of the mixed fabric, on the other hand, a padding technique was used with a padding liquid prepared in a similar manner as mentioned above. The fabric was padded with a 70 % pickup, then subjected to hot-air drying at 110° C for 3 minutes and finally cured at 180° C for 1 minute. The testing fabric subjected to the antimicrobial processing treatment was cut into a 2 cm × 2 cm square. After pressurized and heated in an autoclave, the antimicrobial effect was assayed by a conventional method for determining the number of microbes, using Staphylococcus aureus as the microbes to be determined.

With a view to checking up the fastness of the antimicrobial processing applied to a fiber material, the antimicrobial activity after 10 washings was assayed in terms of the number of microbes by a simple washing method (40° C × 5 minutes - 2 minute dehydration - 5 minute rinse). The results are set forth in the following table.

Table – Effective Concentration and Cost Performance of Anti-
microbial Preparations with respect to Mixed Fabric and
Corrugated Cardboard

| | Fiber processing | | Corrugated board processing | |
|---|---|---|---|---|
| | (1)Amino-glycoside | (2)Quaternary ammonium salt | (1)Amino-glycoside | (2)Quaternary ammonium salt |
| Effective concentration of antimicrobial preparation | 0.001 % | 0.6 % | 0.001 % | 0.2 % |
| Effective Concentration after 10 washings | 0.01 % | 0.6 g | — | — |
| Amount of antimicrobial preparation required per 10ℓ of padding liquid | 0.1 g | 60 g | — | — |
| Amount of antimicrobial preparation required for smearing of 1m³ of corrugated board | — | — | 0.02 g | 4 g |
| Ratio of cost of anti-microbial preparation to commercial price per said stipulated amount | $\frac{1}{10,000}$ | $\frac{6}{100}$ | $\frac{1}{100,000}$ | $\frac{4}{1,000}$ |

In the recipes of the antimicrobial preparations, a significant concentration difference is found and the antibiotic shows an antimicrobial effect in very small amounts. Therefore, the present preparations are improved in terms of cost performance and are greatly useful in industrial fields.

The antimicrobial preparations of such amino-glycosidic antibiotics are industrially useful possibly as antimicrobial antibromics for sanitary processings of fibers, antimicrobial agents for paints, antimicrobial agents for leather, slime removers, water treatment agents, pool disinfectants, antimicrobial agents for chip pulp, antimicrobial agents for rubber, plastics, films, coated cables and resin emulsions and antimicrobial agents for oils and adhesives as well as possibly for cultural assets (pigments and coloring materials), stainproofing (waxes), environmental microbicides (industrial detergents), printing (ink) and packaging materials.

The present preparations are applicable not only for such antimicrobial processing of fibers and corrugated cardboards as mentioned above, but also as removers for slime occurring in paper-making processes, antimicrobial agents for paints, antimicrobial antibromics for removing damages caused by the microbiral putrefaction of metal processing oils (coolants), as will be described later, and so on.

EXAMPLES

The present invention will now be explained specifically but not exclusively with reference to the following examples.

Example 1

For the purpose of imparting an antibromic effect to cotton fabric, it was subjected to antimicrobial processing with ribostamycin sulfate. The antimicrobial preparation was made with 0.001 % of ribostamycin sulfate and 2 % of organosilicone. For the antimicrobial processing of cotton fabric, a cotton cloth of 30 cm × 40 cm was padded with a 70 % pickup. After hot-air drying was carried out at 110° C for 3 minutes, the cloth was cured at 180° C for 1 minute.

The antimicrobial effect durability before and after 10 washings of the cotton cloth subjected to such antimicrobial processing was assayed by the method for determining the number of microbes(a method for the inhibition of microbial growth established by Japan Antimicrobial & Fungicidal Association at the request of the Sanitary Processings Meeting of Fiber Products). Used as the microbes to be assayed was Staphylococcus auerus (IFO 12732).

It has consequently been found that the fastness of cotton fabric to the antimicrobial processing by ribostamycin sulfate is satisfactory and the processed cloth shows a sterilization rate of 99 % or more even after a simple washings method of 10. This suggests that ribostamycin sulfate is satisfactorily useful as an antimicrobial processing agent. From the fact that it produces an effect in very small amounts, it is noted that its cost performance is high. The cotton cloth was proved to produce an antimicrobial effect upon Staphylococcus aureus and the cloth will inhibit the putrefaction of human organic matters derived as by perspiration in wearing and, hence, prevent the emission of foul smells due to puterfaction. In particular, it has been reported that a sweaty smell is attributable to the activity of Staphylococcus aureus.

Example 2

In most cases, slime generated by microbiological factors in paper-making processes occurs mainly on regions where the rate of flow beocmes slow such as, for instance, the inner walls of pipes and chests. Upon solidified, this slimy material makes paper discontinuous and causes dust, thus giving rise to a drop of the productivity of paper or a deterioration of paper quality. The microbicide used as means for preventing and controlling the occurrence of such a slimy material is the so-called slime controller. Microbes are always present at a concentration of about $10^6$/ml in a slurry having a pulp concentration of about 3 % at which slime occurs, viz., the so-called white water. A part of such microbes takes part in the formation of slime, mainly in the form of bacteria.

In the instant example, the following testing was carried out to apply the amino-glycosidic antibiotic to the slime controller. A 3 % pulp liquid from a paper mill, viz., the white water, was sampled out and then poured into 20 conical flasks of 500 ml in volume, each in an amount of 200 ml. Yeast extract was then added to each sample at a concentration of 0.1 %. Added to each of ten flasks of these flasks was kanamycin at a concentration of 0.001 %. The flask was subjected to rotary culture at 30°C and 180 rpm for two months. In the meantime, an addtional 2 ml of a 1 % kanamaycin solution was added every week to each of the ten flasks to which kanamycin had been added. The formation of slime was confirmed by the presence of small, pulp-containing lumps. Changes in the amount of microbes during culture were calculated by the determination of ATP by fluorescent techniques.

In the control flasks to which only the yeast extract was added, the amount of microbes increased from $10^6$/ml on the first day of culture to $10^8$/ml on the second day, which was maintained for a period of 20 days. On the sixty day after the completion of culture, the amount of microbes decreased to about 2.1 × $10^6$/ml. However, slime was found to occur in all the control flasks.

In the test flasks to which the yeast extract and kanamycin were added, the amount of microbes decreased from $10^6$/ml on the first day to 1.6 × $10^3$/ml, which was kept for a period of 35 days. On the sixty day after the completion of culture, however, the amount of microbes was increased to 6.4 × $10^5$/ml. This suggests that the formation of slime by bacterial groups is effectively prevented in the test flasks, and proves that kanamycin is useful as the slime controller.

Example 3

Deteriorations of paints by microbes tend to occur in bathrooms, kitchens, closets and lavatories. In particular, microbes are prone to occur in food processing plants, breweries, fiber processing plants, tabacco processing plants, sugar refineries and beverage plants, offering a grave problem in view of the purification of environment, the prevention of food pollution, etc. As antifire means, emulstion base paints are now employed in a wide range of fields inclusive of exterior and interior finish works. However, emulsions are prone to be deteriorated by microbes. In particular, bacteria tend to grow in polyvinyl acetate latex paints, acryl latex paints, solvent type of alkyd paints, etc., resulting in denaturation, slime formation, discoloration and emission of foul smells.

The amino-glycosidic antibiotics were stuided as to whether or not they were effectively used as an antiseptic for such paints. The paint under test was a latex emulsion paint based on styrene butadiene rubber, and the microbes under test were Staphylococcus aureus (IFO 12732) and Esherichia coli (IFO 3972). Two strains under test were each cultured on a proliferating medium, and 1 ml of the live microbial liquid having a concentration of about $10^6$/ml was inoculated on 20 g of each sample. The samples were divided into test runs containing 0.001 % of kanamycin and control runs containing no kanamycin. The inoculated samples were cultured at 30° C for periods of seven days, one month and two months. After the completion of culture, 1 g of paint under test was collected from each test run, and then diluted with 20 mℓ of a sterilized saline solution. One (1) mℓ of the diluted solution was then poured onto a petri dish, in which 20 mℓ of an agar medium for determining the number of microbes, kept liquid at 45° C, were added and uniformly dispersed. After cooling, culture was carried out at 30° C for 40 days to count the number of live microbes. Each sample was diluted ten times.

As a result, the number of live microbes calculated per 1 g of sample in each control run was found to be $2 \times 10^3$/g on the 7th day, $3 \times 10^4$/g on the first month and $2 \times 10^4$/g on the second month for Staphylococcus aureus. For live Esherichia coli, its number was $1.2 \times 10^4$/g on the 7th day, $2 \times 10^5$/g on the first month and $4 \times 10^4$/g on the second month.

In each test run in which kanamycin was added, the number of microbes was equal to or less than $10^2$/g, the figure clearly indicating an effect upon the inhibition of microbial proliferation. This suggests the fact that kanamycin is chemically coupled to the paint under test, thereby sustaining its antimicrobial activity, and proves that kanamycin is usefully used as an antiseptic for emulsion paints.


Example 4

Where any microbiological control is not applied to a metal processing oil (coolant) run and operated in an open system, microbes optimized to that environment grow preferentially. If available conditions are well-arranged, then the microbes proliferate explosively, giving rise to various troubles. Upon undergoing the decompositional action of microbes, the composition of the coolant is so ill-balanced that its performance may drop, while difficulty may often be encountered in the maintenance of quality of the product, partly because the product rusts. This results in frequent replacement of liquid, which has an adverse influence upon productivity from the economical viewpoint.

Of more importance are unpleasant smells of trimethylamine, dimethylamine, etc. emitted in the process of microbial proliferation (comprising a complicated combination of volatile basic nitrogen compounds, methyl mercaptan, sulfur compounds such as hydrogen sulfide, organic acids such as lactic acid and caproic acid, esters and the like). As the criteria for the replacement of oils first stipulte that they should be replaced "when foul smells are emitted by promoted putrefaction", it has been reported that the reasons for oil replacement are mostly attributable to the putrefaction of microbes. Thus, a microbial problem is of vital importance in metal processing sites. To apply the amino-glycosidic antibiotic as an antimicrobial antibromic for such metal processing oils, the following experimentation was carried out.

Antiseptic experiments for metal processing oils were performed according to JIS K 2241-1980. One (1) % of corn powders and rusted iron swarfs were put in a 250 mℓ wide-mouthed bottle, to which given concentrations of reagents under test were added to prepare 100 ml of liquids under test. In a control run, no reagent under test was added. Thus prepared liquids were inoculated with 1 % microbes under test every five days. (To this end, five microbes Bacillus subtilis ATCC 15442, Escherichia coli IFO 3972, Pseudomonas aeruginosa ATCC 15442, Pseudomonas fluroescens ATCC 13525, Proteus vulgaris ATCC 13315 were cultured on proliferating liquid media to a concentration of $10^7$/mℓ or more). Such inoculation was repeated 10 times at a culture temperature of 30° C. The concetrations of the reagents in the liquids under test were set at 0.1 %, 0.01 %, 0.001 % and 0.0001 %. For the reagents, kanamycin was used. The assay of the antiseptic effect was made in terms of increases in the number of microbes, hue, pH reductions, viscosity, oixdation-reduction potentials and putrid smells.

As a result, the test runs, in which 0.001 % and 0.0001 % of kanamycin were added, showed as much an increase in the number of microbes as the control run and a pH reduced to 6 or less, but showed no sign of putrid smells. On the other hand, the test runs, in which 0.1 % and 0.01 % of kanamycin were added, were lower in the increase in the number of microbes than the control run, but showed as much an increase in the number of microbes as the control run after the 20th day.

After the completion of testing, the liquids under test showed a pH of about 7 with no emission of putrid smells. As a result of identification of the microbes in the liquids after culture, Pseudomonas aeruginosa and Pseudomonas fluorescens were preferentialy found in the 0.1 % and 0.01 % test runs, and the presence of Escherichia coli in addition to said two microbes was confirmed in the 0.001 % test run.

From this fact, it has been suggested that kanamycin has a limited antimicrobial effect on two Pseudomonas microbes. From the fact that any putrid smell was not emitted, it has been presumed that no noticeable denaturation of the coolant takes place. Since the amino-glycoside has no substantial influence upon metal processing, it is believed that it can be well used as an antiseptic for metal processing oils, if studies are made of how to use it.

## Claims

Application of an amino-glycosidic antibiotic to an antimicrobial/antibromic processing preparation, in which said amino-glycosidic antibiotic is applied as antimicrobial agents for sanitary processings of fibers, antimicrobial agents for paints, antimicrobial agents for leather, antimicrobial antibromic agents for slime controllers for paper mills and for paper boards, antimicrobial agents for rubber, plastics, films, coated cables and resin emulsions, antimicrobial agents for oils and adhesives, antimicrobial agents for printing and antimicrobial agents for packaging materials.

## DOCUMENTS CONSIDERED TO BE RELEVANT

| Category | Citation of document with indication, where appropriate, of relevant passages | Relevant to claim | CLASSIFICATION OF THE APPLICATION (Int Cl.⁴) |
|---|---|---|---|
| X | HANS-JÜRGEN REHM "Industrielle Mikrobiologie" second edition, 1980, SPRINGER-VERLAG, Berlin, Heidelberg, New York pages 691-703 * Page 692, line 27 - page 693, line 24; especially page 693, lines 6,7,17-21 * -- | 1 | A 01 N 63/00<br>D 06 M 13/355<br>C 09 D 5/14<br>C 09 D 5/16<br>D 21 H 21/36<br>A 61 L 15/46 |
| X | GB - A - 1 521 171 (THE UNIVERSITY OF STRATHCLYDE) * Page 1, lines 54-57 * -- | 1 | |
| X | DEVELOPEMENTS IN INDUSTRIAL MICROBIOLOGY, vol. 1, 1960, Plenum Press, New York J.C.CHAPIN: "Problems in the selection, testing, and formulation of antibacterials to be used as textile additives." pages 59-64 * Page 59, lines 1-8 * -- | 1 | |
| A | DE - A - 2 139 261 (E.R.SQUIBB & SONS INC.) * Page 4, lines 9-23 * -- | 1 | TECHNICAL FIELDS SEARCHED (Int Cl.⁴)<br><br>A 01 N<br>D 06 M<br>C 09 D<br>D 21 H<br>A 61 L |
| A | US - A - 3 728 213 (HINZ) * Abstract * -- | 1 | |
| A | US - A - 4 289 581 (KATAYAMA et al.) * Abstract * -- | 1 | |
| A | US - A - 4 290 846 (MUNTWYLER) | 1 | |

The present search report has been drawn up for all claims

| Place of search | Date of completion of the search | Examiner |
|---|---|---|
| VIENNA | 22-05-1990 | SCHNASS |

## EUROPEAN SEARCH REPORT

**European Patent Office**

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| | * Column 2, line 39 – column 3, line 9 * <br> ---- | | |
| | | | TECHNICAL FIELDS SEARCHED (Int. Cl.4) |

The present search report has been drawn up for all claims

| Place of search | Date of completion of the search | Examiner |
|---|---|---|
| VIENNA | 22-05-1990 | SCHNASS |